Europäisches Patentamt

(19) European Patent Office

Office européen des brevets

(11) Veröffentlichungsnummer: **0 014 457**
**B1**

(12) # EUROPÄISCHE PATENTSCHRIFT

(45) Veröffentlichungstag der Patentschrift:
**17.11.82**

(51) Int. Cl.³: **C 07 D 301/10, B 01 J 23/96**

(21) Anmeldenummer: **80100560.4**

(22) Anmeldetag: **04.02.80**

(54) **Verfahren zur Herstellung und Regenerierung von Trägerkatalysatoren sowie deren Verwendung für die Herstellung von Äthylenoxid.**

(30) Priorität: **09.02.79 DE 2904919**

(43) Veröffentlichungstag der Anmeldung:
**20.08.80 Patentblatt 80/17**

(45) Bekanntmachung des Hinweises auf die Patenterteilung:
**17.11.82 Patentblatt 82/46**

(84) Benannte Vertragsstaaten:
**BE DE FR GB IT NL**

(56) Entgegenhaltungen:
**EP-A-0 001 078**
**DE-A-2 209 392**
**DE-B-2 519 599**
**US-A-3 692 136**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft,**
**Carl-Bosch-Strasse 38, D-6700 Ludwigshafen (DE)**

(72) Erfinder: **Mross, Wolf Dieter, Dr., Londoner Ring 75,**
**D-6700 Ludwigshafen (DE)**
Erfinder: **Titzenthaler, Eckart Dr., Lenbachstrasse 11,**
**D-6700 Ludwigshafen (DE)**
Erfinder: **Koopmann, Juergen, Dr.,**
**Walter-Bruch-Strasse 48, D-6730 Neustadt 11 (DE)**
Erfinder: **Vogt, Volker, Dr., Rieslingweg 8,**
**D-6706 Wachenheim (DE)**
Erfinder: **Schwarzmann, Matthias, Dr.,**
**Carl-Bosch-Strasse 54, D-6703 Limburgerhof (DE)**

# 0 014 457

### Verfahren zur Herstellung und Regenerierung von Trägerkatalysatoren
### sowie deren Verwendung für die Herstellung von Äthylenoxid

Die vorliegende Erfindung betrifft ein neues Verfahren zur Herstellung und Regenerierung von Silber und Alkalimetalle enthaltenden Trägerkatalysatoren mit verbesserter Wirkung für die Herstellung von Äthylenoxid aus Äthylen und Sauerstoff in der Gasphase.

Derartige Katalysatoren sind, sieht man von der erfindungsgemäßen Verbesserung ab, allgemein bekannt. Sie enthalten als aktive Komponenten Silber und Alkalimetalle und werden in der Weise hergestellt, daß man das Trägermaterial, z. B. Aluminiumoxid, mit einer Silber- und Alkalimetall enthaltenden Lösung imprägniert und anschließend erhitzt, wobei das Silber in die aktive metallische Form übergeht.

Aus der DE-B-2 519 599 ist es bekannt, daß die Wirkung von Silber enthaltenden Äthylenoxid-Katalysatoren, die nach längerer Betriebszeit in ihrer Selektivität bezüglich des Äthylenoxids nachgelassen haben, verbessert werden kann, wenn man ein schweres Alkalimetallkation wie Rubidium und insbesondere Caesium auf sie aufbringt. Eine ähnliche Verbesserung erzielt man gemäß der US-A-4 033 903 mit ungebrauchten, jedoch thermisch vorbehandelten Äthylenoxid-Katalysatoren. Bei ungebrauchten oder thermisch nicht vorbehandelten Katalysatoren bewirkt eine in üblicher Weise vorgenommene nachträgliche Dotierung mit den schweren Alkalimetallen indes nur eine geringe Selektivitätsverbesserung. Wenn man die schweren Alkalimetalle gleichzeitig mit dem Silber auf das Trägermaterial aufbringt, wird die Selektivität zwar ebenfalls verbessert, jedoch nimmt diese mit Gebrauch des Katalysators verhältnismäßig schnell wieder ab. Die leichten Alkalimetalle, welche vornehmlich zur Erhöhung der Standfestigkeit dienen, d. h. welche die volle Wirksamkeit der Katalysatoren verlängern, können hingegen zusammen mit dem Silber auf das Trägermaterial aufgebracht werden.

Der Erfindung lag daher die allgemeine Aufgabe zugrunde, die aktivierende Wirkung der schweren Alkalimetalle auch für ungebrauchte Katalysatoren nutzbar zu machen sowie daneben auch die Regenerierung im Hinblick auf das Ergebnis zu verbessern. Diese Aufgabe wurde bereits befriedigend gemäß einem älteren Vorschlag (siehe EP-A-0 002 045) gelöst, indem man die schweren Alkalimetalle mittels einer Lösung auf den bereits Silber und gegebenenfalls die leichten Alkalimetalle enthaltenden Trägerkatalysatoren aufbringt, die eine sauerstoff- oder stickstoffhaltige Verbindung enthält, welche mit Ag(I)-Ionen Komplexsalze zu bilden vermag.

In Weiterverfolgung dieser allgemeinen Aufgabe war es speziell die Aufgabe der vorliegenden Erfindung, die Dotierung der Katalysatoren mit den schweren Alkalimetallen und damit die Wirksamkeit der Katalysatoren weiter zu verbessern.

Dementsprechend wurde ein Verfahren zur Herstellung und Regenerierung von Silber auf Alkalimetalle enthaltenden Trägerkatalysatoren für die Herstellung von Äthylenoxid aus Äthylen und Sauerstoff in der Gasphase durch Dotierung eines neuen oder gebrauchten Trägerkatalysators, der bereits Silber in metallischer Form sowie gegebenenfalls die leichten Alkalimetalle Lithium und/oder Natrium und daneben auch, falls es sich um einen bereits gebrauchten Katalysator handelt, die schweren Alkalimetalle Kalium, Rubidium und/oder Caesium enthält, mit einer Lösung, welche die schweren Alkalimetalle enthält, gefunden, welches dadurch gekennzeichnet ist, daß diese Dotierungslösung

a)  im Falle der Herstellung eines neuen Katalysators ein oberflächenaktives Mittel und/oder ein Reduktionsmittel und
b)  im Falle der Regenerierung eines gebrauchten Katalysators ein oberflächenaktives Mittel

enthält.

Es wurde weiterhin gefunden, daß die Wirkung dieser Mittel weiter erhöht werden kann, wenn man sowohl im Falle (a) als auch im Falle (b) zusätzlich eine sauerstoff- oder stickstoffhaltige Verbindung in der Dotierungslösung mitverwendet, welche mit Ag(I)-Ionen Komplexverbindungen bildet.

Das Verfahren beruht auf der Hypothese und damit zusammenhängenden Beobachtungen, daß für die Wirksamkeit des Katalysators eine räumliche Wechselwirkung zwischen dem metallischen Silber einerseits und den Alkalimetallkationen andererseits gewährleistet werden muß. Während diese Wechselwirkung im Falle der leichten Alkalimetalle offensichtlich gegen äußere Einflüsse verhältnismäßig unempfindlich ist, hängt sie im Falle der schweren Alkalimetalle vermutlich davon ab, daß die schweren Alkalimetallkationen am metallischen Silber nur haften, wenn sie sich möglichst nahe hieran befinden und wenn keine Silberkationen mehr zugegen sind, welche aufgrund ihrer naturgemäß größeren Affinität zum metallischen Silber für eine räumliche Trennung der Alkalimetallkationen vom Silber und damit für eine Verminderung der Wechselwirkung verantwortlich sind. Dies steht im Einklang mit den erfindungsgemäßen Maßnahmen, denn die Reduktionsmittel und die komplexbildenden Mittel bewirken eine Herabsetzung der Ag-Ionen-Konzentration, und die oberflächenaktiven Mittel bewirken eine verbesserte Benetzung des Silbers mit der Dotierungslösung.

Die erfindungsgemäßen Katalysatoren werden also in zwei Stufen hergestellt, wobei die erste Stufe die Herstellung des Grundkatalysators I und die zweite die Herstellung des mit den schweren Alkalimetallen dotierten fertigen Katalysators II betrifft.

Für den Grundkatalysator und dessen Herstellung gelten die Vorschriften des Standes der Technik, d. h. man imprägniert das Trägermaterial mit einer Lösung, welche Silbersalze und gegebenenfalls Lithium und/oder Natriumsalze enthält und erhitzt das imprägnierte Material auf 150 bis 300°C, wobei das Silber in die metallische Form überführt wird.

Der Grundkatalysator ist hierbei vorzugsweise durch die Kriterien von Tabelle 1 gekennzeichnet.

Tabelle 1

Zusammensetzung des Grundkatalysators I in Gew.-%

| Trägermaterial | rd. 88—98, | vorzugsweise 88—94% |
|---|---|---|
| Silber | rd. 2—12, | vorzugsweise 6—12% |
| $Li^+ + Na^+$ | 0,005—0,03, | vorzugsweise 0,005—0,025% |
| $Li^+$ | 0,005—0,025, | vorzugsweise 0,005—0,015% |
| $Na^+$ | 0,0015—0,035, | vorzugsweise 0,004—0,025% |
| Atomverhältnis (abgerundet): | | |
| Ag: $(Li^+ + Na^+)$ | 5 : 1—1500 :1, | vorzugsweise 15 : 1—150 : 1 |

Als Trägermaterial kommen u. a. Silikate, Quarz, Siliciumcarbid und Graphit in Betracht, besonders aber $\alpha$-Aluminiumoxid. Als Raumform für den Träger eignen sich wie allgemein für Gasphasenreaktionen an Festbettkontakten z. B. Kugeln und vor allem Ringe mit einem Durchmesser von 5 bis 10 mm.

Zur Imprägnierung setzt man das Silber vorzugsweise in Form von Amin-Komplexsalzen ein, welche als Anionen Nitrat, Carbonat, Hydroxid oder die Anionen von Carbonsäuren enthalten. Als Amin-Komponente eignen sich beispielsweise Alkyl- und Hydroxyalkylamine mit etwa bis zu 6 C-Atomen. Gut bewährt haben sich u. a. die Butylamine, darunter vor allem das sec.-Butylamin. Die leichten Alkalimetalle verwendet man vorzugsweise in Form von Salzen mit den obengenannten Anionen, und als Lösungsmittel dient bevorzugt Wasser.

Die Zusammensetzung der Imprägnierlösung richtet sich, wie durch Vorversuche einfach zu ermitteln ist, nach dem Aufnahmevermögen des Trägermaterials sowie nach dem gewünschten Gehalt der aktiven Katalysatorbestandteile im fertigen Katalysator. Erforderlichenfalls kann die Imprägnierung jeweils nach Zwischentrocknung mehrfach wiederholt werden. Ist die vorgegebene Menge der aktiven Katalysatorbestandteile auf den Träger aufgebracht, so erhitzt man ihn wie üblich auf 150 bis 300°C. Obwohl das Erhitzen in nicht-oxidierender Atmosphäre an sich zweckmäßig wäre, nimmt man es aus praktischen Gründen im Luftstrom vor, zumal die vollständige Reduktion des Silbers durch die erfindungsgemäße Nachbehandlung gewährleistet wird. Hiernach ist der Grundkatalysator I gebrauchsfertig. Die mit diesen Katalysatoren erzielbaren, auf einen Sauerstoff-Umsatz von 50% bezogenen Selektivitäten betragen in aller Regel 65 bis 75%.

Dotiert man den Katalysator I erfindungsgemäß zusätzlich mit 0,0005—0,025, vorzugsweise mit 0,005—0,025 Gew.-%, bezogen auf dessen Gesamtmenge, mit einem schweren Alkalimetall, also mit Kalium und/oder Rubidium und/oder vor allem Caesium, so erzielt man hierdurch eine Selektivitätsverbesserung von etwa bis zu 15 Prozentpunkten.

Die erfindungsgemäßen Maßnahmen bewirken einerseits eine deutliche Selektivitätsverbesserung auch bei ungebrauchten Katalysatoren I sowie eine entsprechende Verbesserung hinsichtlich der hohen Selektivitäten bei gebrauchten Katalysatoren I und II.

Zur Bereitung der dotierten Katalysatoren II geht man von üblichen, vorzugsweise alkoholischen Lösungen von Verbindungen der schweren Alkalimetalle aus, mit denen man den Katalysator I bzw. den gebrauchten Katalysator II imprägniert. Derartige Verbindungen sind vorzugsweise die Hydroxide und Nitrate; daneben eignen sich aber auch die Carbonate sowie die Salze von Carbonsäuren. Als Lösungsmittel eignen sich besonders die $C_1 - C_3$-Alkanole.

Die für die nachbehandelnde Imprägnierung der Katalysatoren I erforderliche Menge der Lösung richtet sich nach der Flüssigkeitsaufnahme und ist durch Vorversuche unschwer zu ermitteln. Im allgemeinen beträgt diese Menge 100—500 ml pro Kilogramm des Katalysators. Ihr Gehalt an schweren Alkalimetallkationen beträgt etwa 0,005—0,15, vorzugsweise 0,015—0,1 Gew.-%, wobei die Konzentrationen im unteren Bereich für den Fall einer wiederholten Regenerierung gelten, d. h. für den Fall, daß der zu behandelnde Katalysator bereits die schweren Alkalimetalle enthält.

Die Menge der schweren Alkalimetalle im fertigen Katalysator II beträgt vorzugsweise 0,0005—0,025, insbesondere 0,005—0,025 Gew.-%.

Die erfindungsgemäß zu verwendenden oberflächenaktiven Mittel entfalten ihre gewünschte Wirksamkeit bereits in sehr niedrigen Konzentrationen, jedoch verwendet man sie im allgemeinen in Konzentrationen von 0,01—10, vorzugsweise 0,1—5 Gew.-% der Imprägnierlösung.

Prinzipiell eignen sich jegliche schwefel- und halogenfreie Netzmittel, jedoch werden nicht-ionische Tenside bevorzugt. Solche sind u. a. allgemein Polyglykoläther (Äthoxylierungsprodukte) von aliphatischen, cycloaliphatischen und alkylaromatischen Alkoholen sowie Fettsäureester und Fettsäureamide. Als Beispiele seien die handelsüblichen Tenside Ölsäureäthanolamid, der Monostearinsäureester des Triäthanolamins, das Umsetzungsprodukt von Nonylphenol und 14 Mol Äthylenoxid und das Umsetzungsprodukt von Oleylamin und 12 Mol Äthylenoxid genannt.

Die erfindungsgemäß zu verwendenden Reduktionsmittel haben die Funktion, Silberkationen zu reduzieren. Man setzt sie daher in gleichen oder leicht überschüssigen molaren Mengen ein, wie der Silberionenkonzentrationen im Katalysator I entspricht. Diese Mengen liegen in aller Regel bei 0,5—10 Gew.-% der Imprägnierlösung. Die Ag-Ionen-Konzentration im Katalysator kann in der Weise bestimmt werden, daß man die Ionen mit Wasser eluiert und ihre Menge anschließend titrimetrisch bestimmt.

Wegen der leichten Reduzierbarkeit der Silber-Ionen eignen sich praktisch alle Reduktionsmittel mit Ausnahme von denjenigen, die Halogen oder Schwefel enthalten, da diese den Katalysator vergiften können. Als Reduktionsmittel seien beispielsweise genannt: Hydrazin, Hydrochinon, p-Aminophenol, p-Diaminobenzol und p-Hydroxy-N-methylanilin.

Zusätzlich zu den oberflächenaktiven Mitteln und/oder zu den Reduktionsmitteln kann sich die Mitverwendung von solchen Verbindungen empfehlen, welche mit Ag-I-Ionen Komplexverbindungen bilden. Solche Komplexbildner sind beispielsweise Diketone wie Diacetyl und Acetylaceton, Acetessigester, mehrfache Äther wie die sogenannten Kronenäther, Ammoniak, Nitrile und Amine wie vorzugsweise aliphatische und cycloaliphatische Amine. Unter den stickstoffhaltigen Komplexbildnern seien z. B. Acetonitril, Mono-, Di- und Trialkyl- und Trialkanolamine mit insgesamt bis zu 6 C-Atomen, Äthylendiamin und Piperazin genannt.

Man verwendet diese Komplexbildner in den Dotierungslösungen zweckmäßigerweise in Konzentrationen von 0,1—30, vorzugsweise 0,1—10 Gew.-%.

Die Dotierung mit den Lösungen, welche die schweren Alkalimetalle und die erfindungsgemäß zuzusetzenden Mittel enthalten, erfolgt wie üblich durch Imprägnieren der Katalysatoren I. Handelt es sich um eine Regenerierung, so läßt man die Lösungen über den fest angeordneten Kontakt laufen und bläst das überschüssige Lösungsmittel z. B. mit Stickstoff ab. Danach trocknet man den Katalysator und erhitzt ihn allmählich unter Stickstoff auf 150—300°C. Der so behandelte Katalysator ist sodann betriebsbereit.

Die Herstellung des Äthylenoxids aus Äthylen und Sauerstoff in der Gasphase erfolgt unter üblichen Bedingungen, also im Molverhältnis Äthylen : $O_2$ von 0,5—5 : 1, bei einem Druck von 1—50 bar und einer Temperatur von 150—350°C, gegebenenfalls unter Mitverwendung von inerten Gasen und Inhibitoren. Nähere Angaben hierzu erübrigen sich daher, zumal die erfindungsgemäße Herstellung der Katalysatoren in allen Fällen eine Verbesserung mit sich bringt.

Diese Verbesserung besteht vornehmlich in einer Selektivitätserhöhung bezüglich des Äthylenoxids, und zwar unabhängig davon, ob leichte Alkalimetalle zugegen sind oder nicht. In den meisten Fällen bewirken die leichten Alkalimetalle eine zusätzliche Erhöhung der Selektivität, besonders aber der Standzeit.

Ein weiterer Vorteil der erfindungsgemäß mit den Reduktionsmitteln hergestellten Katalysatoren II liegt darin, daß sie in der Anfahrphase ihres Gebrauchs im Gegensatz zu herkömmlichen Katalysatoren kein ausgeprägtes Aktivitätsmaximum aufweisen. Dies bedeutet, daß sich die zur Vermeidung einer nicht mehr unter Kontrolle zu haltenden exothermen Reaktion erforderliche exakte schwierige Temperaturführung in der Anfahrphase erübrigt oder daß diese Temperaturführung wesentlich erleichtert wird.

## Beispiel 1

### Herstellung eines Grundkatalysators I

1 kg handelsübliches α-Aluminiumoxid wurde mit einer Lösung aus 120 g sec.-Butylamin, 29 g Wasser, 139 g Silbernitrat und 1,39 g Lithiumnitrat imprägniert und anschließend in einem Umluftofen bei 220°C getrocknet. Dieser Katalysator enthielt 8,0 Gew.-% Silber und 0,015 Gew.-% Lithium (Li+).

Das α-Aluminiumoxid der Firma Norton, Typ SA 5551, hatte die Form von Ringen (Durchmesser 7,5 mm, Dicke 2,5 mm) eine Wasseradsorption von 0,23 ml/g, eine Oberfläche von 0,23 m²/g und eine Schüttdichte von 1,1 g/ml.

# 0 014 457

## Beispiel 2

### Herstellung verschiedener Katalysatoren II

Je 100 g des Grundkatalysators gemäß Beispiel 1 wurden mit einer Lösung aus 16,2 g Methanol, 16,4 mg Caesiumhydroxid und 0,5 g eines oberflächenaktiven Mittels imprägniert und anschließend bei 200°C im Umluftofen getrocknet. Dieser Katalysator enthielt neben den Bestandteilen des Grundkatalysators 0,015 Gew.-% Caesium (Cs+).
Die Art der oberflächenaktiven Mittel ist der Tabelle 2 zu entnehmen.

## Beispiel 3

### Herstellung von Äthylenoxid

Die Katalysatoren gemäß den Beispielen 1 und 2 wurden auf eine Teilchengröße von 0,5 bis 0,6 mm zerkleinert und in einen Testreaktor (Katalysatorfüllung 5 g) gefüllt. Durch diesen Kontakt wurde bei 15 bar und T°C ein Gasgemisch aus 8 Vol.-% Sauerstoff, 30 Vol.-% Äthylen, 1 ppm Vinylchlorid und dem Rest Stickstoff geleitet. Die Temperatur T wurde so eingestellt, daß jeweils ein Sauerstoffumsatz von 50% erzielt wurde. Die Selektivitäten S bezüglich des Äthylenoxids sind der Tabelle 2 zu entnehmen.

Tabelle 2

| Vers. Nr. | Katalysator | Oberflächenakt. Mittel | T °C | S % |
|---|---|---|---|---|
| 1 V*) | I, Beisp. 1 | — | 218 | 72,0 |
| 2 V*) | II, Beisp. 2 | — | 219 | 75,0 |
| 1 | II/1 | Nonylphenol + 14 Äthylenoxid | 219 | 81,0 |
| 2 | II/2 | Kokosfettalkohol + 8 Äthylenoxid | 218 | 81,5 |
| 3 | II/3 | Nonylphenol + 8 Äthylenoxid | 223 | 82,0 |
| 4 | II/4 | Oleylamin + 12 Äthylenoxid | 220 | 82,0 |
| 5 | II/5 | Nonylphenol + 12 Äthylenoxid | 221 | 81,5 |
| 6 | II/6 | Triäthanolaminmonostearat | 224 | 82,0 |
| 7 | II/7 | Talgfettalkohol + 20 Äthylenoxid | 222 | 81,0 |
| 8 | II/8 | Ölsäureäthanolamid | 221 | 82,0 |

*) Zum Vergleich.

## Beispiel 4

### Herstellung eines Grundkatalysators I

Dieser Katalysator wurde analog Beispiel 1 hergestellt, jedoch nicht so lange getrocknet, so daß das Ag+ nur zu 88,7% reduziert wurde. Der Rest lag in ionischer Form vor.

## Beispiel 5

### Herstellung verschiedener Katalysatoren II

Je 100 g des Grundkatalysators gemäß Beispiel 4 wurden mit einer Lösung aus 16,2 g Methanol, 16,4 mg Caesiumhydroxid, 0,5 g eines Reduktionsmittels, 0,5 g eines oberflächenaktiven Mittels und

5

0,5 g eines Komplexbildners imprägniert und anschließend bei 200°C im Umluftofen getrocknet. Dieser Katalysator enthielt neben den Bestandteilen des Grundkatalysators 0,015 Gew.-% Caesium (Cs+).

Die Art der Mittel ist der Tabelle 3 zu entnehmen.

### Beispiel 6

### Herstellung von Äthylenoxid

Die Herstellung des Äthylenoxids erfolgte mit den Katalysatoren des Beispiels 5 analog Beispiel 3.

Die Ergebnisse sind aus Tabelle 3 ersichtlich. Bei den Vergleichsversuchen 1V und 2V mußte die Temperatur in der Anfahrphase wegen des Aktivitätsmaximums um etwa 20°C zurückgenommen und danach allmählich wieder erhöht werden. Bei den Versuchen 1 bis 15 war diese Maßnahme hingegen nicht erforderlich.

Tabelle 3

| Versuch Nr. | Katalysator | Reduktionsmittel | Oberflächenaktives Mittel | Komplexbildner | T °C | S % |
|---|---|---|---|---|---|---|
| 1 V*) | I, Beisp. 4 | — | — | — | 218 | 69,0 |
| 2 V*) | II, Beisp. 5 | — | — | — | 218 | 71,0 |
| 1 | II/1**) | Hydrazin | — | Octylamin; | 218 | 82,0 |
| 2 | II/2 | Hydrazin | — | sec.-Butylamin; | 218 | 82,5 |
| 3 | II/3 | Hydrazin | — | sec.-Butylamin; | 222 | 82,5 |
| 4 | II/4 | p-Aminophenol | — | Dodecylamin; | 225 | 82,0 |
| 5 | II/5 | p-Diaminobenzol | — | Laurylamin; | 223 | 81,5 |
| 6 | II/6 | p-N-Methylaminophenol | — | sec.-Butylamin; | 222 | 82,0 |
| 7 | II/7 | p-Phenyl-3-pyrazolidon | — | Propylamin; | 225 | 81,5 |
| 8 | II/8 | H₂O₂ | — | sec.-Butylamin; | 221 | 80,0 |
| 9 | II/9 | p-Diaminobenzol | Nonylphenol + 14 ÄO | — | 226 | 82,5 |
| 10 | II/10 | Hydrochinon + p-Aminophenol 1 : 1 | — | sec.-Butylamin; | 222 | 82,5 |
| 11 | II/11 | Hydrazin | Nonylphenol + 8 ÄO | — | 222 | 82,0 |
| 12 | II/12 | Hydrazin | Nonylphenol + 12 ÄO | — | 225 | 82,5 |
| 13 | II/13 | Hydrazin | Nonylphenol + 8 ÄO | sec. Butylamin | 223 | 82,5 |
| 14 | II/14 | Hydrochinon | Ölsäureäthanolamid | Octylamin | 224 | 82,5 |
| 15 | II/15 | p-Aminophenol | Oleylamin + 12 ÄO | Dodecylamin | 226 | 82,0 |

*) Zum Vergleich;
**) Jeweils gemäß Beispiel 5;
ÄO = Äthylenoxid.

0 014 457

**Patentansprüche**

1.Verfahren zur Herstellung und Regenerierung von Silber und Alkalimetalle enthaltenden Trägerkatalysatoren für die Herstellung von Äthylenoxid aus Äthylen und Sauerstoff in der Gasphase durch Dotierung eines neuen oder gebrauchten Trägerkatalysators, der bereits Silber in metallischer Form sowie gegebenenfalls die leichten Alkalimetalle Lithium und/oder Natrium und daneben auch, falls es sich um einen gebrauchten Katalysator handelt, die schweren Alkalimetalle Kalium, Rubidium und/oder Caesium enthält, mit einer Lösung, welche die schweren Alkalimetalle enthält, dadurch gekennzeichnet, daß diese Dotierungslösung

a) im Falle der Herstellung eines neuen Katalysators ein oberflächenaktives Mittel und/oder ein Reduktionsmittel und

b) im Falle der Regenerierung eines gebrauchten Katalysators ein oberflächenaktives Mittel

enthält.

2. Verfahren nach Anspruch 1, dadurch gekennzeichnet, daß man sowohl im Falle (a) als auch im Falle (b) zusätzlich ein Mittel mitverwendet, welches mit Ag-I-Ionen Komplexe bildet.

3. Verfahren nach den Ansprüchen 1 und 2, dadurch gekennzeichnet, daß man es auf die Herstellung von Katalysatoren anwendet, die durch folgende Zusammensetzung gekennzeichnet sind

| | |
|---|---|
| rd. 88 – 98 Gew.-% | Trägermaterial |
| rd. 2 – 12 Gew.-% | Silber |
| 0,0005 – 0,3 Gew.-% | Lithium und/oder Natrium |
| 0,0005 – 0,025 Gew.-% | Kalium und/oder |
| | Rubidium und/oder Caesium |

4. Verfahren nach den Ansprüchen 1 bis 3, dadurch gekennzeichnet, daß man als Trägermaterial $\alpha$-Aluminiumoxid verwendet.

5. Verfahren nach den Ansprüchen 1 bis 4, dadurch gekennzeichnet, daß das schwere Alkalimetall Caesium ist.

6. Verfahren zur Herstellung von Äthylenoxid aus Äthylen und Sauerstoff in der Gasphase bei 1 bis 50 bar und 150 bis 350° C, dadurch gekennzeichnet, daß man hierzu die gemäß den Ansprüchen 1 bis 5 hergestellten Katalysatoren verwendet.

**Claims**

1. A process for the preparation and regeneration of supported catalysts containing silver and alkali metals, for the preparation of ethylene oxide from ethylene and oxygen in the gas phase, by modifying a new or spent supported catalyst which already contains metallic silver and may or may not contain the light alkali metals lithium and/or sodium and, where a spent catalyst is concerned, also contains the heavy alkali metals potassium, rubidium and/or cesium, with a solution containing the heavy alkali metals, characterized in that this modifier solution contains

a) a surfactant and/or reducing agent, in the case of the preparation of a new catalyst, or

b) a surfactant, in the case of the regeneration of a spent catalyst.

2. A process as claimed in claim 1, characterized in that, both in case (a) and in case (b), an agent which forms complexes with Ag(I) ions is used additionally.

3. A process as claimed in claims 1 and 2, characterized in that it is used for the preparation of catalysts which are characterized by the following composition:

| | |
|---|---|
| about 88 – 98% | by weight of carrier |
| about 2 – 12% | by weight of silver |
| 0.0005 – 0.3% | by weight of lithium and/or sodium |
| 0.0005 – 0.025% | by weight of potassium and/or rubidium and/or cesium. |

4. A process as claimed in claims 1 to 3, characterized in that $\alpha$-alumina is used as the carrier.

5. A process as claimed in claims 1 to 4, characterized in that the heavy alkali metal is cesium.

6. A process for the preparation of ethylene oxide from ethylene and oxygen in the gas phase at from 1 to 50 bar and from 150 to 350° C, characterized in that a catalyst prepared by the process of any of claims 1 to 5 is used.

# 0 014 457

## Revendications

1. Procédé pour la préparation et la régénération de catalyseurs à l'argent et aux métaux alcalins sur support, pour la préparation d'oxyde d'éthylène en phase gazeuse à partir de l'éthylène et de l'oxygène, par dopage d'un catalyseur sur support frais ou usé, qui contient déjà de l'argent dans sa forme métallique, ainsi que le cas échéant des métaux alcalins légers lithium et(ou) sodium et, s'il s'agit d'un catalyseur usé, des métaux alcalins lourds potassium, rubidium et(ou) césium, à l'aide d'une solution contenant des métaux alcalins lourds, caractérisé en ce que la solution de dopage renferme:

a) dans le cas d'une préparation d'un catalyseur frais un produit tensio-actif et(ou) un agent réducteur,
b) dans le cas d'une régénération d'un catalyseur usé un produit tensio-actif.

2. Procédé suivant la revendication 1, caractérisé en ce que, dans le cas a) aussi bien que dans le cas b), on emploie en outre une substance complexant les ions Ag-I.

3. Procédé suivant les revendications 1 et 2, caractérisé en ce qu'il est mis en oeuvre pour la préparation de catalyseurs de la composition ci-après:

| | |
|---|---|
| matière support | environ 88 à 98% en poids |
| argent | environ 2 à 12% en poids |
| lithium et(ou) sodium | 0,0005 à 0,3% en poids |
| potassium et(ou) rubidium et(ou) césium | 0,0005 à 0,025% en poids. |

4. Procédé suivant les revendications 1 à 3, caractérisé en ce que l'on emploie comme matière support de l'oxyde d'aluminium $\alpha$.

5. Procédé suivant les revendications 1 à 4, caractérisé en ce que le métal alcalin lourd est le césium.

6. Procédé de préparation d'oxyde d'oxyde d'éthylène à partir de l'éthylène et de l'oxygène en phase gazeuse entre 150 et 350°C sous 1 à 50 bars, caractérisé en ce que l'on emploie des catalyseurs préparés par le procédé suivant les revendications 1 à 5.

9